# EUROPEAN PATENT SPECIFICATION

(11) **EP 3 590 360 B1**
(45) Date of publication and mention of the grant of the patent: **15.10.2025**
(21) Application number: 18841084.9
(22) Date of filing: 01.08.2018
(51) Int. Cl.: A23L 33/105, A23L 2/39, A23L 2/52, A23L 5/44, A61P 9/00, A61P 27/06, A61K 31/05, A61K 36/00

(54) **LUTEIN-CONTAINING COMPOSITION AND METHOD FOR MANUFACTURING SAME**
LUTEINHALTIGE ZUSAMMENSETZUNG UND VERFAHREN ZUR HERSTELLUNG DAVON
COMPOSITION CONTENANT DE LA LUTÉINE ET PROCÉDÉ POUR LA PRODUIRE

(30) Priority: 02.08.2017 JP 2017150110
(43) Date of publication of application: 08.01.2020
(73) Proprietor: Petroeuroasia Co., Ltd., Suntou-gun, Shizuoka 411-0903 (JP)
(72) Inventor: TAKAHASHI Hidehiro, Suntou-gun Shizuoka 411-0907 (JP)
(74) Representative: Regimbeau
(86) International application number: PCT/JP2018/028896
(87) International publication number: WO 2019/026970

(56) References cited:
- WO-A1-2014/033703
- JP-A- 2008 094 806
- JP-A- 2015 528 292
- JP-B2- 3 302 999
- US-A1- 2010 034 956
- TIPPEL JANINE ET AL: "Colour stability of lutein esters in liquid and spray dried delivery systems based on Quillaja saponins", FOOD RESEARCH INTERNATIONAL, ELSEVIER, AMSTERDAM, NL, vol. 87, 16 June 2016 (2016-06-16), pages 68 - 75, XP029671196, ISSN: 0963-9969, DOI: 10.1016/J.FOODRES.2016.06.014
- FANCL, JAPAN: "Freshly Squeezed Green Juice with Soy Powder Sticks", GNPD, June 2013 (2013-06-01), pages 1 - 4, XP009518606, Retrieved from the Internet <URL:https://www.gnpd.com/sinatra/recordpage/2054666/> [retrieved on 20181017]
- TIPPEL, J. ET AL.: "Colour stability of lutein esters in liquid and spray dried delivery systems based on Quillaja saponins", FOOD RESEARCH INTERNATIONAL, vol. 87, 2016, pages 68 - 75, XP029671196

## Description

### CROSS-REFERENCE TO RELATED APPLICATIONS

This application enjoys the benefits of priority from preceding Japanese Patent Application No. 2017-150110 (filing date: August 2, 2017).

### TECHNICAL FIELD

The present invention relates to a lutein-containing composition and a method of producing the same, namely to a water-dispersible powder composition containing lutein and a method of producing the same.

### BACKGROUND ART

Lutein is a type of carotenoids contained in green and yellow vegetables, egg yolks, animal fats and the like, and is known to exert antioxidant activity *in vivo.* Taking lutein aggressively thus has been expected to bring a preventive effect against diseases (e.g., hypertension, cancer, angina pectoris, myocardial infarction, hepatitis) referred to as so-called lifestyle-related diseases in which it is considered that active oxygen is involved *in vivo.* Since lutein is present in the macular area of eyes and can absorb high-energy light, such as ultraviolet light or blue light, and can also erase active oxygen generated by the light, lutein is considered to prevent retinal degeneration and maintain ocular health.

Carotenoids, including lutein, are, however, substances with poor water solubility. For practical use of lutein as foods or the like, solubilization of lutein into water has been the problem. So far, a technique in which carotenoids, such as lutein, are microencapsulated by protective coating (Patent Document 1) and a technique in which xanthophylls, such as lutein, are combined with egg-yolk phospholipids, thus causing the resultant composition to be water-dispersible (Patent Document 2) have been reported.

### Reference List

### Patent Documents

[Patent Document 1] JP 2005-517694 A
[Patent Document 2] JP 2008-127505 A

It is known from e.g. US2010/034956 to stabilize a dried lutein composition with *inter alia* quillaja saponin.

### SUMMARY OF INVENTION

An objective of the present invention is to provide a water-dispersible, lutein-containing composition and a method of producing the same.

The present inventor has found currently that a powder composition obtained by performing homogenization process of lutein, which is poor-water-solubility substance, with a saponin to obtain an emulsion composition, followed by drying of the obtained emulsion composition, is water-dispersible and possesses an excellent stability after dissolution. The present inventor has further found that, even after preserved, the obtained powder composition excels in water solubility and in stability after dissolution. This result is surprising because despite lutein's poor water solubility, the obtained lutein-containing composition can be completely dissolved into water, and moreover the solution is stable. The present inventor has yet further found that to mix the powder composition with an antioxidant can prevent lutein from discoloring and decomposing. The present invention is based on these findings.

According to the present invention, the following inventions are provided:
[1] A water-dispersible powder composition, comprising: (A) lutein, (B) a quillaja saponin, and (E) an antioxidant which is ascorbic acid or a salt or ester thereof.
[2] The composition according to [1], wherein a mixing ratio (by mass based on solid content) of saponin to lutein is 0.06 or more.
[4] The composition according to any one of [1] to [3], further comprising: (C) an excipient and/or (D) a thickener.
[6] A food comprising the composition according to any one of [1] to [5].
[7] The food according to [6], wherein the food is in beverage form.
[8] A method of producing the composition according to any one of [1] to [5], comprising the steps of:
   performing homogenization process of (A), (B) and (E) in water to obtain an emulsion composition; and
   drying the obtained composition.

According to the present invention, lutein, which is poor-water-solubility substance, can easily be dispersed in water by mixing it with the saponin, leading to an excellent stability after dissolution as well. The present invention is therefore advantageous in that it can facilitate use of lutein as foods (especially, beverages) and the like. Further, since saponins are approved for food use and are not allergen, it is also advantageous in respect that the composition of the present invention can be taken without anxiety.

### DETAILED DESCRIPTION OF THE INVENTION

A composition of the present invention comprises (A) lutein, (B) a quillaja saponin and (E) an antioxidant which is ascorbic acid or its salt or ester.

### (A) Lutein

Lutein, called β, ε-carotene-3,3'-diol, is a type of carotenoids. In the present invention, lutein shall include, unless otherwise stated, not only a free-form lutein, but also an ester-form lutein, such as fatty acid esters. Preferably, the lutein is a free-form lutein.

Sources of the lutein used in the present invention are not limited to particular ones, and the lutein extracted from plants, animals, algae, microorganisms and the like, which may be purified as necessary, may be used, or alternatively the lutein produced by synthesis or commercially available may be used.

The lutein content (in terms of solid content) in the composition of the present invention can be 5 to 35% by mass, preferably 15 to 25% by mass. Since the lutein content of the composition of the present invention can be set to 20% or more by mass while maintaining dispersibility of lutein to water, the composition of the present invention is advantageous in that it can supply more amount of lutein to foods and the like even if supplying the same additive amount of lutein.

### (B) Saponins

Saponins are glycosides in which a glucose binds to a steroid or triterpene, which are present in plants and have foaming property. Classifying saponins based on their sources, for example, saponins include Quillaja saponin, Yucca saponin, soy saponin, carrot saponin and the like. In the present invention, Quillaja saponins that are roughly purified or purified from plants may be used. In the present invention, for example, a quillaja extract can be used as Quillaja saponin.

In the present invention, it is preferred that Quillaja saponin be used alone, or alternatively Quillaja saponin be used in combination with another saponin.

The saponin content (in terms of solid content) in the composition of the present invention can be an amount that enables lutein to be dispersible to water, e.g., can be 0.5 to 15.0% by mass, preferably 1.0 to 10.0% by mass.

Regarding the mixing ratio (by mass based on solid content) of saponin to lutein, the lower limit can be 0.06 (preferably 0.08, more preferably 0.10, still more preferably 0.12) in light of enhancement of the solubility to water of lutein. Regarding the mixing ratio (by mass based on solid content) of saponin to lutein, the upper limit can also be 1.25 (preferably 0.88, more preferably 0.63, still more preferably 0.38) in consideration of influence of bitter taste and the like of saponins. The mixing ratio (by mass based on solid content) of saponin to lutein can be, for example, 0.06 to 1.25 (preferably 0.12 to 0.63).

A method for measuring the lutein and saponin contents is not particularly limited, and they can be measured by, e.g., gas chromatography, HPLC.

The composition of the present invention may further comprise one or two or more components selected from the group consisting of (C) an excipient, (D) a thickener.

### (C) Excipient

In the present invention, one or two or more excipients can be used to provide the composition of the present invention in powder form. As excipients that can be used for the present invention, any excipients that are acceptable for foods and the like may be used, and examples of excipients include starches, processed starches, saccharides, celluloses. Among them, examples of starches include corn starch, wheat starch, potato starch, and examples of processed starches include dextrin, maltodextrin, powdered corn syrup, solubilized starch. Examples of saccharides include sugar alcohols (e.g., xylitol, maltitol, inositol) in addition to monosaccharides and disaccharides (e.g., lactose, xylose). In the present invention, dextrin and/or maltodextrin can be preferably used as the excipient.

The excipient content (in terms of solid content) in the composition of the present invention can be 5 to 60% by mass, preferably 10 to 50% by mass.

### (D) Thickener

In the present invention, one or two or more thickeners can be used to provide the composition of the present invention in powder form. As thickeners that can be used for the present invention, any thickeners that are acceptable for foods and the like may be used, and examples of thickeners include gum arabic, arabinogalactan, alginic acid, welan gum, elemi resin, artemisia sphaerocephala seed gum, curdlan, cassia gum, sodium caseinate, carrageenan, karaya gum, carob bean gum, xanthan gum, chitin, chitosan, guar gum, glucosamine, psyllium seed gum, gellan gum, tamarind gum, *Hibiscus manihot,* tragacanth gum, *Bacillus natto* gum, furcellaran, pullulan, pectin, Macrophomopsis gum, methylcellulose, *Colpomenia sinuosa* extract. In the present invention, gum arabic can be preferably used as the thickener.

The thickener content (in terms of solid content) in the composition of the present invention can be 0.1 to 20% by mass, preferably 2 to 15% by mass.

### (E) Antioxidant

In the present invention, at least one antioxidant is used to suppress oxidation and decomposition of lutein contained in the composition of the present invention. As antioxidant, ascorbic acid and ester and salt thereof is used. Examples of the ascorbic acid and ester and salt thereof include L-ascorbic acid, L-ascorbic acid ester, sodium L-ascorbate, L-ascorbyl palmitate. In the present invention, the ascorbic acids and/or salt thereof can be preferably used as the antioxidant.

The antioxidant content (in terms of solid content) in the composition of the present invention can be 0 to 30% by mass, preferably 2 to 25% by mass.

To the composition of the present invention, any components that are acceptable for foods and the like other than the foregoing (A), (B), (C), (D) and (E) may be added, and such components include flavors, preservatives.

The composition of the present invention can be produced by homogenization process of raw materials as described later. Accordingly, the composition of the present invention can include the components (A), (B) and (E) above and optionally, the components (C) and (D) above and any other components, in a homogenized state.

The composition of the present invention is also an emulsified composition obtained by drying an emulsified composition of lutein, which is water-dispersible. Herein, whether or not a composition is "water-dispersible" can be evaluated by visual observation of a solution state obtained by adding 0.2 g of the composition (in terms of solid content) into 100 mL of water at room temperature and then stirring and mixing the mixture. Specifically, when the total amount of the composition having been added is dissolved in water and no suspensions nor precipitates is generated, the composition can be determined as "water-dispersible."

Since the composition of the present invention is a water-dispersible, lutein-containing composition, lutein can be added, as a raw material, to a food to produce the food in which the lutein is mixed homogeneously. The composition of the present invention also can be added, as a raw material, not only to a food but also to a feed or pet food to produce the feed and pet food in which the lutein is mixed homogeneously. That is, the present invention can provide a food, feed and pet food in which the composition of the present invention is mixed.

The food into which the composition of the present invention can be mixed is not particularly limited and includes waters (e.g., drinking water, hydrogen water, mineral water, near water), soft, refreshing liquids (e.g., fruit juice, vegetable juice, drinks including fruit and vegetable juice, carbonated drinks, functional drinks, sports drinks, non-alcoholic drinks), tea drinks (e.g., green tea, black tea, Chinese teas, roasted-barley tea, buckwheat tea, mate, blended tea), coffee drinks (e.g., canned coffee, instant coffee), cocoa drinks, lactic acid bacteria drinks, Japanese Aojiru, soymilk drinks, dairy products (e.g., milk, milk beverage, processed milk, yogurt), energy drinks, jelly drinks, soups, alcoholic drinks (e.g., beer, low-malt beer, whiskey, bourbon, spirits, liqueur, wine, fruit wine, Sake, Shochu, other miscellaneous liquors), carbohydrate-containing foods (e.g., rice, noodles, breads, pasta), western-style confectionery (e.g., biscuit, cracker, cookie, waffle, pie), Japanese-style confectionery (e.g., rice cracker, sweetened bean paste bun, adzuki-bean jelly), chewing gums, candies, frozen desserts (e.g., ice cream, and sherbet).

The food into which the composition of the present invention can be mixed also may be in liquid form, such as drink, fluid diet, may be in paste, semiliquid, or gel form, or may be in solid or powder form. Since the composition of the present invention excels not only in solubility to water but also in stability after dissolved into water, it is advantageous in that the food into which the composition of the present invention is mixed can be provided in beverage form despite of containing lutein, which is poor-water-solubility.

A mixing amount of the composition of the present invention in the food of the present invention can be determined using a daily intake of lutein as a rough target. A daily effective intake of lutein for human in cases where its antioxidant activity is desired is 1 to 50 mg (preferably, 5 to 15 mg), and the composition of the present invention can be mixed into the food of the present invention such that such a daily effective intake of lutein for human can be taken. In this case, the food of the present invention may be packaged in such a way that the daily effective intake of lutein can be taken. The food may be in package form of a single serve or of being divided into multiple packages as long as the daily effective intake can be taken. In cases where lutein is provided in the package form, it is desirable that statements for the intake be printed on the package such that the daily effective intake can be taken, or the package be provided together with a package insert including such statements. Further, in cases where the daily effective intake is provided in the multiple packages, for convenience in intake, a set of the multiple packages including the daily effective intake can be provided.

The composition of the present invention can be produced by performing homogenization process of raw materials and dry process of the obtained emulsion composition.

The homogenization process of raw materials can be performed by first adding the raw materials including lutein and quillaja saponin into water and mixing the mixture, further adding the ascorbic acid on antioxidant to the mixture and mixing the mixture, and optionally further adding an excipient and/or thickener to the mixture and mixing the mixture, followed by homogenization process of the obtained mixture.

The homogenization process is a process for reducing size of component particles included in the mixture and homogenizing the particles. The homogenization process can be performed with a homogenizer (e.g., Milder (manufactured by Pacific Machinery & Engineering Co., Ltd.), Silverson homogenizer (manufactured by SILVERSON NIPPON CO., LTD.)), high-pressure homogenizer (e.g., Homogenizer (manufactured by SANWA MACHINE CO., INC.), High-pressure homogenizer (manufactured by SANMARU MACHINERY CO., LTD.,)) or the like. In case of using homogenizers, the homogenization can be performed under the condition of 25 to 40 m/s. In case of using high-pressure homogenizers, the homogenization can be performed under the condition of 50 to 150 MPa, and may be performed with further strong force being applied. By the homogenization process, the lutein-containing emulsion composition can be obtained. In the emulsion composition, the lutein is in a homogenized state, i.e., in a state where it is dispersed in water.

The dry process of the emulsion composition can be performed by a known method, such as spray drying, freeze drying. By the dry process, a powder lutein-containing composition can be obtained, and the obtained powder lutein-containing composition includes lutein with being homogenized with other components.

### EXAMPLES

The present invention will now be more specifically described based on the following examples. It is noted that examples 1 and 3 are regarded as illustrative and do not form part of the claimed invention.

### Example 1: Production of lutein-containing powder composition in which Quillaja saponin is mixed

According to the mixing amount in Table 1, the lutein-containing powder compositions in which Quillaja saponin was mixed were produced. Specifically, Quillaja saponin was dissolved into 500 mL of water and heated to 65°C, and then to the resultant solution, lutein was added and mixed. To the mixture, dextrin and gum arabic were added and dissolved. After the dissolution, the mixture was emulsified using a high-pressure homogenizer (manufactured by SANMARU MACHINERY CO., LTD., 500 kg/cm², repeated three times). After the emulsification, using a spray dryer (Mini Spray Dryer GB22, manufactured by Yamato Scientific Co., Ltd.), lutein dry powder in which Quillaja saponin was mixed was prepared.

**[Table 1]**

| Table 1: Lutein-containine powder composition in which Quillaja saponin is mixed | |
|---|---|
| Experimental section | Mixed components |
| 1 | Lutein: 100 g |
| | Quillaja saponin: 5 g (50 g of Quillajanin S-100 manufactured by MARUZEN PHARMACEUTICALS CO., LTD.) |
| | Dextrin: 150 g |
| | Gum arabic: 30 g |
| 2 | Lutein: 100 g |
| | Quillaja saponin: 10 g (100 g of Quillajanin S-100 manufactured by MARUZEN PHARMACEUTICALS CO., LTD.) |
| | Dextrin: 150 g |
| | Gum arabic: 30 g |
| 3 | Lutein: 100 g |
| | Quillaja saponin: 20 g (200 g of Quillajanin S-100 manufactured by MARUZEN PHARMACEUTICALS CO., LTD.) |
| | Dextrin: 150 g |
| | Gum arabic: 30 g |
| 4 | Lutein: 100 g |
| | Quillaja saponin: 30 g (300 g of Quillajanin S-100 manufactured by MARUZEN PHARMACEUTICALS CO., LTD.) |
| | Dextrin: 150 g |
| | Gum arabic: 30 g |

| | |
|---|---|
| Lutein: manufactured by PRAKRUTI PRODUCTS PVT., LTD. (India), of free-form, 80% of purity Dextrin: manufactured by San-ei Sucrochemical Co., Ltd. Gum arabic: manufactured by NIPPON FUNMATSU YAKUHIN Co., LTD. | |

For the obtained lutein dry powder, state of the powder, solubility to water, and stability after dissolved and then settled for eight hours were evaluated. The state of the powder and the solubility to water were classified into three levels (A: good, B: normal, C: bad) and evaluated through visual observation. The solubility to water was also observed after addition of 0.2 g of the lutein dry powder into 100 mL of water at room temperature and stirring and mixing the mixture.

The results are as shown in Table 2.

**[Table 2]**

| Table 2: Evaluation for lutein-containing powder composition in which Quillaja saponin is mixed | | | |
|---|---|---|---|
| Experimental section | State of powder | Solubility to water | Stability after settled for 8 hrs |
| 1 | A | A | Suspensions were slightly generated. |
| 2 | A | A | The state where the composition had been dissolved was maintained. |
| 3 | A | A | The state where the composition had been dissolved was maintained. |
| 4 | A | A | The state where the composition had been dissolved was maintained. |

Furthermore, the obtained lutein dry powder was transferred by 10 g to individual glass bottles, each of which was stored at 55°C. On the 14th day, state of the powder, solubility to water, stability after dissolved and then settled for eight hours, and the remaining amount of lutein were evaluated. The solubility to water was classified into three levels (A: good, B: normal, C: bad) and evaluated through visual observation. The solubility to water was also observed after addition of 0.2 g of the lutein dry powder into 100 mL of water at room temperature and stirring and mixing the mixture.

The results are as shown in Table 3.

**[Table 3]**

| Table 3: Preservation stability of lutein-containine powder composition in which Quillaja saponin is mixed | | | | |
|---|---|---|---|---|
| Experimental section | State of powder | Solubility to water | Stability after settled for 8 hrs | Remaining amount of lutein |
| 1 | Discoloration | A | Precipitates were generated. | 64% |
| 2 | Discoloration | A | The state where the composition had been dissolved was maintained. | 72% |
| 3 | Discoloration | A | The state where the composition had been dissolved was maintained. | 70% |
| 4 | Discoloration | A | The state where the composition had been dissolved was maintained. | 69% |

As described above, the lutein-containing powder compositions in which Quillaja saponin is mixed have excelled not only in solubility to water but also in stability after the dissolution. The lutein-containing powder compositions in which Quillaja saponin is mixed have also excelled not only in solubility to water after preservation but also in stability after the dissolution, but oxidation of lutein due to the preservation has been found.

### Example 2: Production of lutein-containing powder composition in which antioxidant is mixed

According to the mixing amount in Table 4, the lutein-containing powder compositions in which an antioxidant was mixed were produced. Specifically, Quillaja saponin was dissolved into 500 mL of water and heated to 65°C, and then to the resultant solution, lutein was added and mixed. To the mixture, sodium ascorbate, dextrin and gum arabic were added and dissolved. After the dissolution, the mixture was emulsified using a high-pressure homogenizer (manufactured by SANMARU MACHINERY CO., LTD., 500 kg/cm², repeated three times). After the emulsification, using a spray dryer (Mini Spray Dryer GB22, manufactured by Yamato Scientific Co., Ltd.), lutein dry powder in which the antioxidant was mixed was prepared.

**[Table 4]**

| Table 4: Lutein-containing powder composition in which antioxidant is mixed | |
|---|---|
| Experimental section | Mixed components |
| 2 | Lutein: 100 g |
| | Quillaja saponin: 10 g (100 g of Quillajanin S-100 manufactured by MARUZEN PHARMACEUTICALS CO., LTD.) |
| | Dextrin: 150 g |
| | Gum arabic: 30 g |
| 5 | Lutein: 100 g |
| | Quillaja saponin: 10 g (100 g of Quillajanin S-100 manufactured by MARUZEN PHARMACEUTICALS CO., LTD.) |
| | Dextrin: 150 g |
| | Gum arabic: 30 g |
| | Sodium Ascorbate: 10 g |
| 6 | Lutein: 100 g |
| | Quillaja saponin: 10 g (100 g of Quillajanin S-100 manufactured by MARUZEN PHARMACEUTICALS CO., LTD.) |
| | Dextrin: 150 g |
| | Gum arabic: 30 g |
| | Sodium Ascorbate: 25 g |
| 7 | Lutein: 100 g |
| | Quillaja saponin: 10 g (100 g of Quillajanin S-100 manufactured by MARUZEN PHARMACEUTICALS CO., LTD.) |
| | Dextrin: 150 g |
| | Gum arabic: 30 g |
| | Sodium Ascorbate: 50 g |
| 8 | Lutein: 100 g |
| | Quillaja saponin: 10 g (100 g of Quillajanin S-100 manufactured by MARUZEN PHARMACEUTICALS CO., LTD.) |
| | Dextrin: 150 g |
| | Gum arabic: 30 g |
| | Sodium Ascorbate: 75 g |

| | |
|---|---|
| Lutein: manufactured by PRAKRUTI PRODUCTS PVT., LTD. (India), of free-form, 80% of purity Dextrin: manufactured by San-ei Sucrochemical Co., Ltd. Gum arabic: manufactured by NIPPON FUNMATSU YAKUHIN Co., LTD. Sodium Ascorbate: manufactured by Royal DSM | |

The obtained lutein dry powder was transferred by 10 g to individual glass bottles, each of which was stored at 55°C. On the 14th day, state of the powder, solubility to water, stability after dissolved and then settled for eight hours, and the remaining amount of lutein were evaluated. The solubility to water was classified into three levels (A: good, B: normal, C: bad) and evaluated through visual observation. The solubility to water was also observed after addition of 0.2 g of the lutein dry powder into 100 mL of water at room temperature and stirring and mixing the mixture.

The results are as shown in Table 5.

**[Table 5]**

| Table 5: Preservation stability of lutein-containine powder composition in which antioxidant is mixed | | | | |
|---|---|---|---|---|
| Experimental section | State of powder | Solubility to water | Stability after settled for 8 hrs | Remaining amount of lutein |
| 2 | Discoloration | A | The state where the composition had been dissolved was maintained. | 65% |
| 5 | Good | A | The state where the composition had been dissolved was maintained. | 84% |
| 6 | Good | A | The state where the composition had been dissolved was maintained. | 96% |
| 7 | Good | A | The state where the composition had been dissolved was maintained. | 97% |
| 8 | Good | A | The state where the composition had been dissolved was maintained. | 97% |

As described above, the lutein-containing powder compositions in which an antioxidant is mixed have a good state of the powder, and the remaining amount of lutein is 80% or more.

### Example 3: Production of lutein-containing powder composition in which Yucca saponin is mixed

According to the mixing amount in Table 6, the lutein-containing powder compositions in which Yucca saponin was mixed were produced. Specifically, Yucca saponin was dissolved into 1000 mL of water and heated to 65°C, and then to the resultant solution, lutein was added and mixed. To the mixture, dextrin and gum arabic were added and dissolved. In Experimental section 10, sodium ascorbate was additionally added to the mixture, and dissolved. After the dissolution, the mixture was emulsified using a high-pressure homogenizer (manufactured by SANMARU MACHINERY CO., LTD., 500 kg/cm², repeated three times). After the emulsification, using a spray dryer (Mini Spray Dryer GB22, manufactured by Yamato Scientific Co., Ltd.), lutein dry powder in which Yucca saponin was mixed was prepared.

**[Table 6]**

| Table 6: Lutein-containing powder composition in which Yucca saponin is mixed | |
|---|---|
| Experimental section | Mixed components |
| 9 | Lutein: 100 g |
| | Yucca saponin: 75 g (150g of Yucca Sarasaponin 50M manufactured by Watahan Trading Co., Ltd.) |
| | Dextrin: 150 g |
| | Gum arabic: 30 g |
| 10 | Lutein: 100 g |
| | Yucca saponin: 75 g (150g of Yucca Sarasaponin 50M manufactured by Watahan Trading Co., Ltd.) |
| | Dextrin: 150 g |
| | Gum arabic: 30 g |
| | Sodium Ascorbate: 50 g |

| | |
|---|---|
| Lutein: manufactured by PRAKRUTI PRODUCTS PVT., LTD. (India), of free-form, 80% of purity Dextrin: manufactured by San-ei Sucrochemical Co., Ltd. Gum arabic: manufactured by NIPPON FUNMATSU YAKUHIN Co., LTD. Sodium Ascorbate: manufactured by Royal DSM | |

The obtained lutein dry powder was transferred by 10 g to individual glass bottles, each of which was stored at 55°C. On the 14th day, state of the powder, solubility to water, stability after dissolved and then settled for eight hours, and the remaining amount of lutein were evaluated. The solubility to water was classified into three levels (A: good, B: normal, C: bad) and evaluated through visual observation. The solubility to water was also observed after addition of 0.2 g of the lutein dry powder into 100 mL of water at room temperature and stirring and mixing the mixture.

The results are as shown in Table 7.

**[Table 7]**

| Table 7: Preservation stability of lutein-containine powder composition in which Yucca saponin is mixed | | | | |
|---|---|---|---|---|
| Experimental section | State of powder | Solubility to water | Stability after settled for 8 hrs | Remaining amount of lutein |
| 9 | Discoloration | A | The state where the composition had been dissolved was maintained. | 57% |
| 10 | Good | A | The state where the composition had been dissolved was maintained. | 92% |

As described above, the lutein-containing powder composition (Experimental section 9) in which Yucca saponin is mixed has excelled in solubility to water after preservation and in stability after the dissolution, but oxidation of lutein due to the preservation has been found. The lutein-containing powder composition (Experimental section 10) in which an antioxidant is additionally mixed has a good state of the powder, and the remaining amount of lutein is 80% or more.

### Example 4: Production of beverage

For a 50 mL of beverage, using mix described in Table 8, a soft beverage (pH 3.4) in which the composition of the present invention was mixed was produced.

**[Table 8]**

| Table 8: Mixed components for beverage | |
|---|---|
| Component | Mixing amount |
| Composition of the present invention (Example | 33 mg (6 mg as lutein) |
| 2, Experimental section 7) | |
| BILBERON-25 (manufactured by TOKIWA Phytochemical Co., Ltd.) | 250 mg (90 mg as anthocyanin) |
| Reduced Maltose Starch Syrup (manufactured by Mitsubishi Shoji Foodtech Co., Ltd.) | 500mg |
| Citric Acid (manufactured by Satuma Kako Co., Ltd.) | 50mg |
| Sodium Ascorbate (manufactured by Royal DSM) | 100mg |
| ShiroQ (manufactured by Petroeuroasia Co., Ltd.) | 75 mg (30 mg as ubiquinol) |

The produced beverage was transferred by 50 mL to individual brown bottles of 50 mL, each of which was heated and sterilized at center temperature of 85°C for 30 minutes. The bottles of beverage were stored at 40°C, and their coloring, taste, and remaining amount of lutein were confirmed in the second week, the first month, and the second month. The results are as shown in Table 9.

**[Table 9]**

| Table 9: Preservation stability of beverage | | | | |
|---|---|---|---|---|
| | Coloring | Taste | Concentration of lutein | Remaining rate |
| At the beginning of preservation | Slightly-whitish purple | Slightly-sour sweetness | 0.121mg/mL | 100% |
| 2nd Week | No changes | No changes | 0.116mg/mL | 96% |
| 1st Month | No changes | No changes | 0.114mg/mL | 94% |
| 2nd Month | No changes | No changes | 0.115mg/mL | 95% |

Even after preservation at 40°C for two months, the produced beverage did not change in coloring and taste, and also maintained almost the same concentration of lutein as the concentration at the beginning of the preservation. Since the preservation stability test at 40°C for two months is equivalent to a preservation stability test at room temperature for two years, it has been indicated that the beverage in which the composition of the present invention is mixed has a high preservation stability in terms of quality and concentration of lutein.

## Claims

1. A water-dispersible powder composition, comprising: (A) lutein, (B) Quillaja saponin, and (E) ascorbic acid or an ester or a salt thereof.

2. The composition according to claim 1, wherein a mixing ratio (by mass based on solid content) of saponin to lutein is 0.06 or more.

3. The composition according to any one of claims 1 to 2, further comprising: (C) an excipient and/or (D) a thickener.

4. A food comprising the composition according to any one of claims 1 to 3.

5. The food according to claim 4, wherein the food is in beverage form.

6. A method of producing the composition according to any one of claims 1 to 3, comprising the steps of:
performing homogenization process of (A) lutein, (B) Quillaja saponin, and (E) ascorbic acid or an ester or a salt thereof in water to obtain an emulsion composition; and
drying the obtained composition.

## Patentansprüche

1. Wasserdispergierbare Pulverzusammensetzung, umfassend: (A) Lutein, (B) Quillaja-Saponin und (E) Ascorbinsäure oder einen Ester oder ein Salz davon.

2. Zusammensetzung nach Anspruch 1, wobei ein Mischungsverhältnis (nach Masse, bezogen auf den Feststoffgehalt) von Saponin zu Lutein 0,06 oder mehr beträgt.

3. Zusammensetzung nach einem der Ansprüche 1 bis 2, ferner umfassend: (C) einen Hilfsstoff und/oder (D) ein Verdickungsmittel.

4. Lebensmittel, das die Zusammensetzung nach einem der Ansprüche 1 bis 3 umfasst.

5. Lebensmittel nach Anspruch 4, wobei das Lebensmittel in Getränkeform vorliegt.

6. Verfahren zur Herstellung der Zusammensetzung nach einem der Ansprüche 1 bis 3, das die folgenden Schritte umfasst:
Durchführen eines Homogenisierungsprozesses von (A) Lutein, (B) Quillaja-Saponin und (E) Ascorbinsäure oder einem Ester oder Salz davon in Wasser, um eine Emulsionszusammensetzung zu erhalten; und
Trocknen der erhaltenen Zusammensetzung.

## Revendications

1. Composition en poudre dispersible dans l'eau, comprenant : (A) de la lutéine, (B) de la saponine de Quillaja, et (E) de l'acide ascorbique ou un ester ou un sel de celui-ci.

2. Composition selon la revendication 1, dans laquelle le rapport de mélange (en masse sur la teneur en solides) de la saponine à la lutéine est de 0,06 ou plus.

3. Composition selon l'une quelconque des revendications 1 à 2, comprenant en outre : (C) un excipient et/ou (D) un épaississant.

4. Aliment comprenant la composition selon l'une quelconque des revendications 1 à 3.

5. Aliment selon la revendication 4, dans lequel l'aliment se présente sous forme de boisson.

6. Procédé de production de la composition selon l'une quelconque des revendications 1 à 3, comprenant les étapes consistant à :
réaliser un processus d'homogénéisation de (A) lutéine, (B) saponine de Quillaja et (E) acide ascorbique ou un ester ou un sel de celui-ci dans de l'eau pour obtenir une composition en émulsion ; et
sécher la composition obtenue.
